Europäisches Patentamt

(19) European Patent Office

Office européen des brevets

(11) Publication number: **0 240 504**
**B1**

(12) # EUROPEAN PATENT SPECIFICATION

(45) Date of publication of patent specification: **14.11.90**

(21) Application number: **86904790.2**

(22) Date of filing: **18.07.86**

(86) International application number:
**PCT/DK86/00083**

(87) International publication number:
**WO 87/00442 29.01.87 Gazette 87/03**

(51) Int. Cl.⁵: **A 61 M 29/02, A 61 M 25/10**

## (54) A DILATATION CATHETER OR A BALLOON CATHETER ASSEMBLY.

(30) Priority: **19.07.85 DK 3315/85**
**26.11.85 US 801715**

(43) Date of publication of application:
**14.10.87 Bulletin 87/42**

(45) Publication of the grant of the patent:
**14.11.90 Bulletin 90/46**

(84) Designated Contracting States:
**AT BE CH DE FR GB IT LI LU NL SE**

(56) References cited:
**EP-A-0 186 267**
**DE-A-2 513 018**
**GB-A-2 130 093**
**US-A-4 327 736**

(73) Proprietor: **Meadox Medicals, Inc.**
**112 Bauer Drive**
**Oakland New Jersey 07436 (US)**

(72) Inventor: **ANDERSEN, Erik**
**5, Ronnens Kvarter**
**Osted DK-4000 0 Roskilde (DK)**
Inventor: **LEONI, Gianni**
**10, Ulvefodvej**
**DK-2670 Greve Strand (DK)**

(74) Representative: **Blatchford, William Michael**
**et al**
**Withers & Rogers 4 Dyer's Buildings Holborn**
**London EC1N 2JT (GB)**

Courier Press, Leamington Spa, England.

## Description

Background of the invention

This invention relates to catheters. More particularly, the invention relates to dilatation catheters which have a shaft having a portion which can be inflated like a balloon. The ballon portion is expanded by supplying fluid under pressure after it is placed at a point in a blood vessel to increase the patency thereof.

The use of balloon catheters for enlarging the diameter of a blood vessel, for example, at a point of stenosis such as is produced by an accumulation of plaque, is a known medical practice. In one such procedure known as percutaneous transluminal coronary angioplasty, a flexible guide wire is first introduced percutaneously into an artery of a patient and is manipulated to arrive at and penetrate the lumen of the occluded portion of a coronary artery while the patient is viewed on an x-ray imaging screen. The guide wire is followed by a guide catheter which is fed along the guide wire to a point in the coronary artery which is just proximal of the occlusion. The dilatation catheter is then sent along the guide wire, within the guide catheter, into the patient's artery and the balloon portion of the catheter is positioned in the occluded portion of the artery.

One dilatation catheter used in this procedure consists of an inner tube, or cannula, which can pass freely along the guide wire. A cathether shaft surrounds the inner tube and has an inner diameter which is somewhat larger than the outer diameter of the inner tube. The distal end of the shaft comprises a flexible balloon portion which is sealed to the distal end of the inner tube and extends rearwards along the inner tube for short distance, where it is sealed to a more rigid shaft tubing. The balloon portion is capable of expansion when fluid under pressure is directed into the space between the shaft and the inner tube, while the rigid portion of the shaft is not.

After the balloon portion of the catheter is correctly positioned, as seen on the imaging screen, a fluid, such as a radiopaque contrast media under pressure, is introduced into the space between the inner tube and the shaft of the catheter so as to cause the balloon portion to expand and to press against the occluding matter on the inside of the blood vessel. The expansion of the balloon is carefully regulated to prevent possible-over-expansion and over-stressing of the wall of the catheter which might cause it to rupture, while yet putting sufficient force on the blood vessel to accomplish the objectives of the procedure. When the desired enlargement of the occluded portion of the artery has been attained, pressure on the inside of the cathether is relieved, the balloon shrinks to its original size and is removed through the guide catheter. In some prior art catheters, lateral or axial shrinkage of the balloon which occurs at the time of deflation can result in undesirable curving of the distal end of the catheter. In GB—A—2 130 093 there is disclosed a dilation catheter comprising a tube of braided material to limit expansion of the balloon.

In a known catheter, for percutaneous transluminal angioplasty, the proximal end of the catheter comprises a mount which receives the proximal ends of the inner tube and of the shaft and seals them in spaced apart relationship, while providing a passageway for supplying fluid under pressure to the space therebetween. A spring within the mount exerts a force on the inner tube in the distal direction, relative to the mount. When the catheter is pressurized, the inner tube moves against the spring to accomodate the decrease in the length of the balloon which occurs when the balloon expands. Upon release of the pressure, the spring returns the inner tube to its distal position, and the diameter of the balloon is reduced to approximately its former diameter to ease removal of the catheter from the blood vessel. The possibility of curvature of the catheter and shift of position during inflation of the balloon are shortcomings of this device.

In catheters for coronary angioplasty a balloon of higher profile than the main shaft are currently in use.

Accordingly, there is a need for a catheter having a smooth profile and wherein the expanded balloon does not change position within the vessel site and which does not curl during inflation.

Summary of the invention

The catheter of the present invention is a coaxial catheter with a flexible inner tubing and an outer tubing of filament-reinforced elastomeric material. An inflatable balloon portion is formed at the distal end of the outer tubing and is anchored to the distal end of the inner tubing. The balloon and other portions of the outer tubing are reinforced by continuous helical filaments which repeatedly cross each other, being divided into segments which exhibit different behaviour when the catheter is internally pressurized. Thus, the balloon portion of the shaft becomes shorter in length when under pressure, while an axially extendible portion of the shaft becomes longer. By suitably balancing the lengths and the angle of the weave of the respective balloon and axially extendible portions of the reinforcing filaments, the changes in length under pressure of these two portions of the shaft are made to offset each other. Thus, corrections in the position of the inner tubing relative to the outer tubing of the catheter are not needed and the balloon portion remains in the desired position in the blood vessel.

In the catheter of the invention, the reinforcing filaments in at least the balloon and the axially extendible portions of the outer tubing take the form of a braid which is embedded or otherwise encased in yielding plastic material. Prior to pressurization of the catheter, the filaments of the balloon portion lie at an angle which is less than a critical angle of 54.73°, relative to the axis of the balloon. The axially extendible portion of the catheter shaft is constructed with a continuing weave of the same filaments, but these filaments lie at an angle which is greater than the critical angle, relative to the axis of the movable portion. The application of internal pressure to the catheter has opposite effects upon these two portions. Under increasing pressure, the diameter of the balloon portion increases until the angle of the filaments in the wall of the balloon reaches the critical angle and then stops. At the same time, the diameter of the axially extendible portion of the outer tubing decreases until the angle of the filaments in the wall of the movable portion reach the critical angle. As the length of the balloon portion decreases, the length of the axially extendible portion of the outer tubing increases and by proper selection of initial diameters and lengths of the two portions, the shortening of the balloon portion is offset. There is no tendency for the balloon to change position. In catheter of the invention, the proximal end of the inner tubing is fixed, relative to the outer tubing of the catheter. There is no need for an adjusting spring or for manipulation of the position of the inner tubing relative to that of the outer tubing.

Accordingly, it is an object of the invention to provide an improved balloon catheter for percutaneous transluminal coronary angioplasty.

It is an object of the present invention to provide a dilatation catheter for use in balloon angioplasty which can be used without risk of shifting of the location of the balloon from a desired position inside a blood vessel as a result of inflation of the catheter.

It is another object of the invention to provide a balloon catheter which does not require re-positioning of the inner tube relative to the shaft of the catheter during inflation.

It is still another object of the invention to provide a balloon catheter which is capable of operation, at body temperature, at pressures of up to 20.265 bar (20 atmospheres).

It is a further object of the invention to provide a balloon catheter which has the outward appearance of tubing of a consistent diameter over its full length.

Still another object of the invention is to reduce the likelihood of kinking of a balloon catheter while in use, while preserving flexibility and the ability of the catheter to transmit force.

Still other objects and advantages of the invention will in part be obvious and will in part be apparent from the specification.

The invention accordingly comprises an article of manufacture possessing the features, the properties, and the relations of elements which will be exemplified in the article hereinafter described, and the scope of the invention will be indicated in the claims.

Brief description of the drawings

For a fuller understanding of the invention, reference is had to the following description, taken in connection with the accompanying drawings in which:

Fig. 1 is a plan view of a balloon catheter showing an attachment for supplying inflating fluid under pressure;

Figs. 2(a) and 2(b) are schematic diagrams of the axial and circumferential stresses produced in the wall of a hollow cylindrical body by internal pressure;

Figs. 3(a) and 3(b) are schematic representations of the reinforcing braid of the invention in the depressurized and pressurized conditions, respectively;

Figs. 4(a), 4(b) and 4(c) are sectional views of the balloon portion, the shaft portions, and of the catheter fitting of the invention;

Figs. 5(a) and 5(b) are schematic diagrams of the balloon and moving portions of the shaft which are useful in the explanation of further features of the invention; and

Fig. 6 is a sectional view of a catheter constructed in accordance with another embodiment of the invention with the proximal end of the balloon portion fixed to a guide wire.

Detailed description of the invention

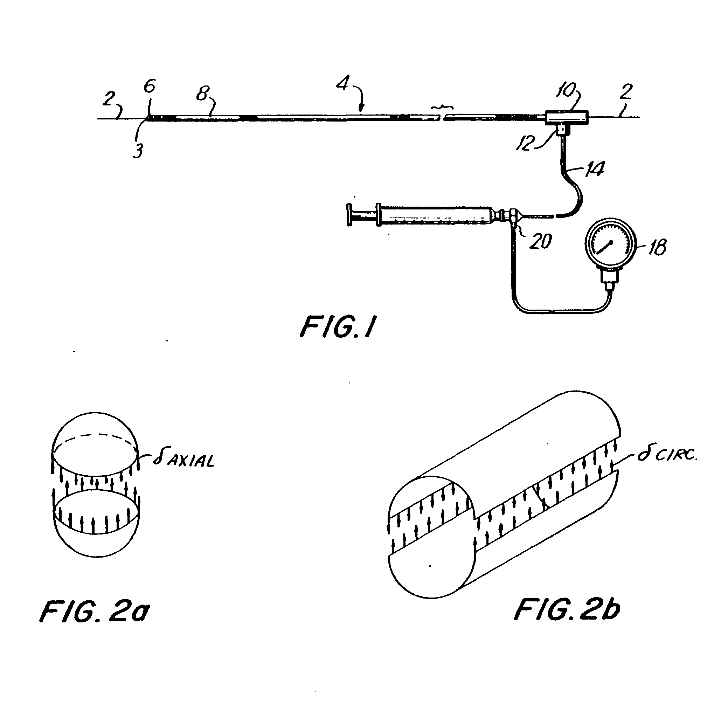

Reference is first made to Fig. 1 in which the location of a guide wire 2 and balloon catheter shaft 4 are shown. The guide wire 2 is, for example, about 0.38 mm (0.015 inches) in diameter and is shown, positioned in the inner passageway 3 of the catheter. The guide wire passes through tip 6 of the catheter, into balloon portion 8, through the remainder of shaft 4 of the catheter, and out through proximal catheter fitting 10. Attached to catheter fitting 10 by means of connecting tee 12 is pressure tubing 14 into which fluid can be forced by means of syringe 16 or other inflation device. Pressure in the fluid can be monitored by means of a gauge 18 which is connected to pressure tubing 14 by means of a second connecting tee 20. The guide catheter which is also conventionally used in placing the balloon catheter in position in a blood vessel is not illustrated.

Reference is now made to Fig. 2 for an exposition of some of the physical principles upon which the invention relies. Figs. 2(a) and 2(b) are respective schematic representations of the axial and circumferential stresses acting within the wall of a hollow cylindrical container which is pressurized internally in the same way as the balloon portion of a catheter axial stress $\delta_{axial}$ is derived as follows:

$$\delta_{axial} \times d \times \pi = Area \times Pressure = \frac{d^2 \times \pi}{4} \times P$$

where d is the diameter of the cylinder. Thus

$$\delta_{axial} = \frac{d \times P}{4}.$$

Similarly, the circumferential stress $\delta_{circ}$ is found from the expression:

$$\delta_{circ} \times L \times 2 = Area \times Pressure \times d \times L \times P,$$

where L equals length. Thus:

$$\delta_{circ} = \frac{L \times P}{2}.$$

The angle of the resultant of the two forces can be expressed as:

$$\tan^2 = \frac{\delta_{circ}}{\delta_{axial}} = 2.$$

The angle α of the resultant vector is thus:

$$\alpha = \tan^{-1}\sqrt{2} = 54.73°.$$

When, therefore, inelastic fibers lying in the plastic walls of a portion of the catheter are at an angle which is equal to the critical angle of 54.73°, relative to the axis, the system is at equilibrium and an increase in the internal pressure of the catheter will result in no change in the catheter diameter. As will be seen, this fact is utilized in the catheter of the invention to limit expansion of the balloon portion as well as to limit the reduction in diameter of a movable portion of the catheter shaft, enabling the accompanying changes in length of these parts under pressure to offset each other.

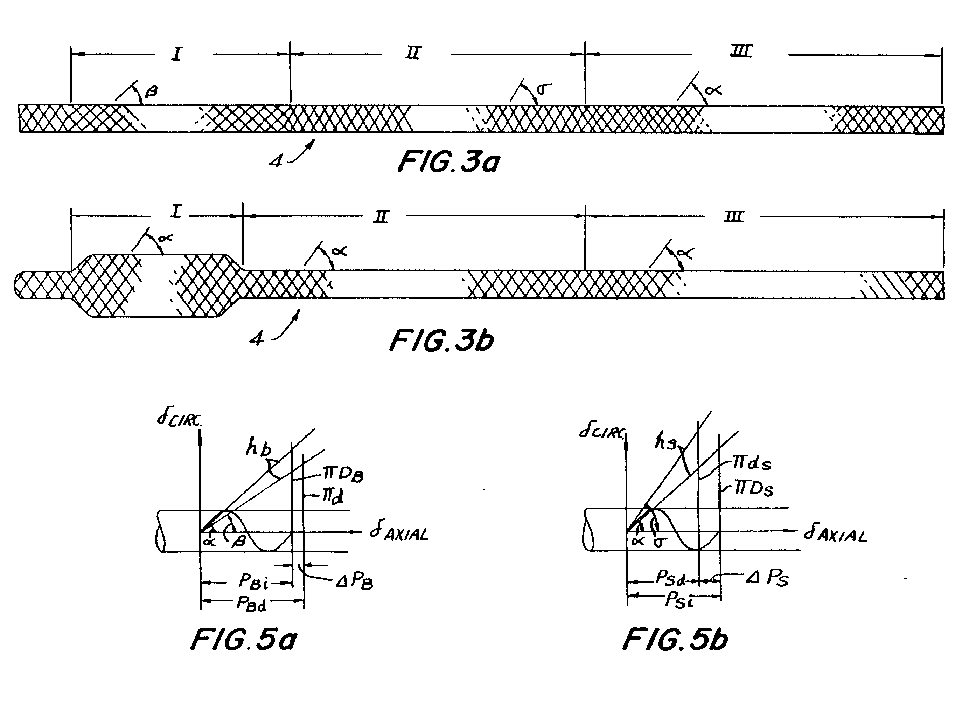

As indicated schematically in Fig. 3(a), the length of catheter shaft 4 of the invention is divided into three portions: a balloon portion I, a movable shaft portion II, and an immovable shaft portion III. Fig. 3(a) shows the orientation of the filaments in the various portions of the catheter shaft prior to the application of pressure to the inside of the catheter. Fig. 3(b) shows the orientation of the same filaments after the application of pressure.

Balloon portion I of the catheter is constructed using braided filaments or fibers lying in helices having an angle β relative to the axis which is less than the critical angle. When the balloon has been pressurized (Fig. 3(b)), the filaments lie in equilibrium at the critical angle and further expansion of the balloon does not occur. The helical filaments in movable portion II of the shaft lie initially at an angle σ which is greater than the critical angle. As pressure is increased, the shaft elongates until the filaments come to rest at the critical angle of 54.73°. Finally, in the immovable portion of the shaft, the filaments have an initial angle which is equal to the critical angle. The dimensions of immovable portion III do not change when the balloon is pressurized.

As shown in Fig. 3(b) after pressurizing, the filaments in each of the three portions of the catheter are all at the critcal angle, e.g., 54.73°, relative to the axis of the system. It will be understood that in a preferred mode of construction the shaft includes continuous helical filaments or fibers of substantially inelastic material which are woven or braided into a reinforcing tube in which the filaments cross each other as they wind along the axis of the tube.

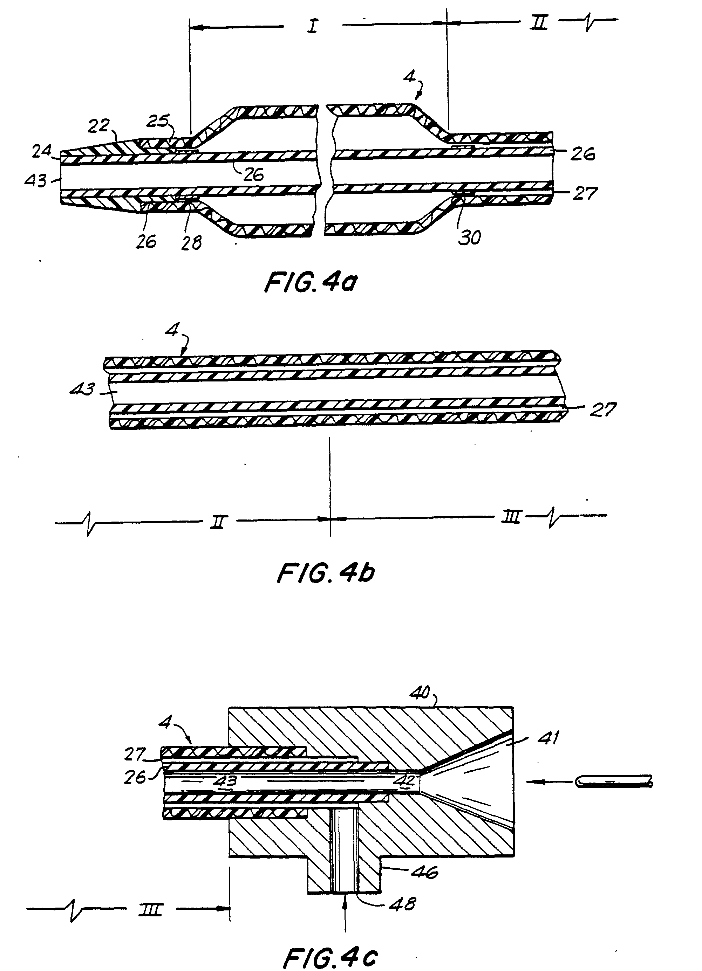

Details of the construction of a working balloon catheter in accordance with the invention are illustrated in the embodiment of Figs. 4(a), 4(b) and 4(c), where Fig. 4(a) shows balloon portion I and the beginning of moving portion II of the shaft. Fig. 4(b) shows the other end of moving portion II and the beginning of immovable shaft portion III. Finally, Fig. 4(c) shows the proximal end of the catheter and illustrates the manner in which the shaft and the inner tube are supported in the connector to receive fluid under pressure.

The distal end 25 of catheter 4 of Fig. 4(a) has a tapered, hollow plastic tip 22 to which distal end 24 of inner catheter tube 26 is sealed. Inner tube 26 is made of a polytetrafluoroethylene plastic having an outside diameter of 0.72 mm and an inside diameter of 0.50 mm through which a guide wire having a diameter of about 0.38 mm (0.015″) may be passed. Neck 26 on distal tip 22 is sealed to the distal end of balloon portion I of shaft 4. As shown in Fig. 4(a) the ballon has been expanded by the application of pressure to the fluid spaced between the inner wall of the catheter shaft 4 and the outer surface of inner tube 26. The length of balloon portion I after inflation is 20 mm; another dimension, as is appropriate for the angioplasty to be performed, may of course be used. The outside diameter of shaft portions II and III (and the unexpanded diameter of balloon portion I) is 1.33 mm, with an inside diameter of 1.00 mm. The expanded diameter of balloon portion I (illustrated in Fig. 4(a)) in the range from 2.5 mm or less up to 10 mm or more may be used, depending upon surgical requirements.

The different portions of the shaft include reinforcing continuous filaments of nylon lying at, respectively, angles of less than critical angle 54.73°, greater than the critical angle, and equal to the critical angle, in a polyurethane body. The polyurethane material may be applied to the filamentary material by dipping or coating the filaments which have been shaped into a tube about a mandrel. The unpressurized lengths of these portions were calculated in the manner to be discussed below.

Also shown in Fig. 4(a) are two bands 28, 30 of radio-opaque material, such as gold or platinum, which are placed around inner tube 26 to mark the ends of balloon portion I so that the balloon can be located radiographically in the blood vessel.

The proximal end of catheter 4 is fastened inside fitting 40 as shown in Fig. 4(c) so as to provide access to the space between inner tube 26 and shaft 5 by fluid under pressure. Fitting 40 includes a central opening 41 for a guide wire which is connected via central passageway 42 to central lumen 43 in inner tube 26 and thence to and out of tip 22.

Both immovable shaft portion III and inner catheter tube 26 are sealed into fitting 40 in spaced-apart, coaxial relationship. Access to the space between the outer surface of inner tube 26 and the inner surface of immovable shaft portion III is provided via passageway 48 in Luer connection 46 which extends laterally from catheter terminal block 40.

The catheter just described was found to be capable of operation at pressures of up to 20.265 bar (20 atmospheres) at a normal body temperature of 37°C. The catheter has the outward appearance of a uniform tubing, with a consistent diameter and has a smooth continuous surface over its full length. It has the lowest profile possible. The catheter does not require a spring as the catheter dip does not move back when balloon inflation occurs. The catheter has the further advantage that, in the event of rupture, the broken portions of the catheter are restrained by the filaments and cannot form an umbrella which would affect removal of the catheter from the patient. Since full length filaments are used in forming the fabric of the outer tubing, the likelihood of kinking of the catheter is reduced, while the catheter remains flexible and preserves its ability to transmit force. At the same time, friction produced during travel of the catheter on the guide wire is minimized.

When the balloon is inflated, movable portion II elongates while the balloon remains in the selected position as it is formed at the distal end of the inner tubing. Since the shaft of the balloon catheter lies in a guiding catheter, the full length of the shaft is available, if needed, to accomodate a change in length which offsets the decrease in length of the balloon portion due to expansion.

The following sets forth the relationship between the structural elements of the various portions of the catheter and provides a basis for calculating dimensions.

First, to find the inflated and deflated pitches of the filamentary spiral in a balloon so that the change in length of the balloon due to expansion can be calculated, it is assumed that the principal filamentary material of which the braid is made will comprise, for example, nylon which exhibits little stretching under tension.

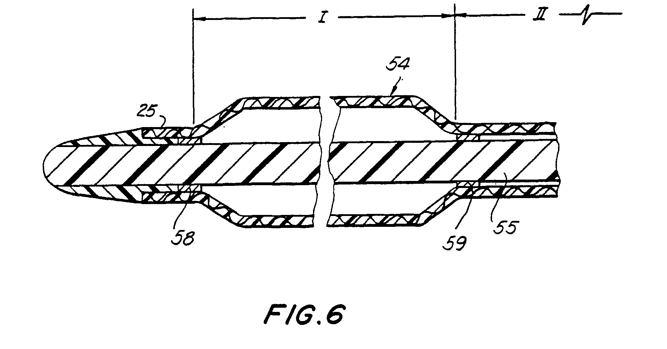

As illustrated in Fig. 5, the pitch $P_{Bi}$ of the inflated balloon is given by:

$$P_{Bi} = \pi D_B / \tan \alpha,$$

where D is the inflated diameter of the balloon portion and $\alpha$ is the critical angle. At equilibrium, the hoop force $\delta_{radial}$ and the axial force $\delta_{axial}$ results in the critical angle $\alpha$ given above, e.g., $\alpha = 54.73°$.

Ths pitch length $P_{Bd}$ of the deflated balloon is given by:

$$P_{Bd} = \pi d_B / \tan \beta,$$

where d is the main diameter of the deflated tube and $\beta$ is the angle of the filaments relative to the axis. Taking the length of the hypotenuse h as constant in the inflated and deflated condition of the balloon, the relationship between the inflated and deflated diameters is found:

$$h_B \sin \alpha = D_B$$
$$h_B \sin \beta = d_B$$

$$\sin \beta = \frac{d_B \sin \alpha}{D_B}.$$

Since $\alpha = 54.73°$, $\sin \alpha = 0.8165$, and:

$$\sin \beta = 0.8165 \frac{d}{D}.$$

Thus, the angle $\beta$ of the filaments in the fabric is determined as a function of the critical angle and the inflated and deflated diameters of the balloon.

The same basic procedure is followed in the calculation of the parameters of the movable shaft, as illustrated in connection with Fig. 5(b).

$$h_s = \frac{D_s}{\sin \alpha} = \frac{d_s}{\sin \sigma},$$

where $h_s$ is the length of the hypotenuse, $D_s$ is the diameter of the inflated shaft, $d_s$ is the diameter of the deflated shaft, $\sigma$ is the angle of the weave in the deflated braid, and as before, $\alpha$ is the critical angle 54.73°.

The two pitch lengths are given by:

$$P_{sd} = \frac{D_s}{\tan \alpha}, \quad P_{si} = \frac{d_s}{\tan \sigma},$$

where $p_{sd}$ is the pitch of the deflated shaft and $P_{si}$ is the pitch of the inflated shaft. The change in length per unit pitch is thus:

$$\Delta P_s = P_{si} - P_{sd}.$$

The number $N_s$ of pitch units required to produce a required change in balloon length $L_B$ is:

$$N_s = \frac{\Delta L_B}{\Delta P_s}$$

and the total movable shaft length $L_s$ can be found:

$$L_s = N_s \times P_{sd}.$$

Since the change in length of the balloon from Fig. 5(a) is:

$$\Delta B = L_{Bd} - L_{Bi},$$

the required pitch number in the movable shaft portion can be calculated. In the working example described above, the length of the catheter was 135 cm, the length of the balloon before pressurizing was about 30 mm and the length of the moving portion of the catheter before pressurizing was approximately 265 mm.

The details of construction of a balloon catheter in accordance with another embodiment of the invention is shown generally as 54 in cross-section in Fig. 6. Catheter 54 includes a balloon portion I and a moving portion II having a distal end 25 as in the case of catheter 4 in Fig. 4a. In this embodiment, distal end 25 of catheter 54 is fixed to the distal end of a guide shaft 55.

Balloon portion I and moving portion II of the shaft of catheter 54 include reinforcing continuous filaments lying at, respectively, angles of less than critical angles 54.73° and greater than the critical angle. Thus, catheter 54 is formed in the same manner as catheter 4. The balloon catheter of Fig. 6 may include radial opaque markers 58 and 59 as in catheter 4 of Fig. 4.

When using a catheter constructed in accordance with this embodiment of the invention, the balloon portion of the catheter is inserted into the vessel at the same time the guide wire is inserted. Thus, introduction of the balloon portion to the selected position is facilitated. Balloon portion I inflates and movable portion II elongates while the balloon remains in the selected position as described in connection with the earlier embodiment.

It will be understood that, to ensure the delivery of ample fluid under pressure to the balloon portion of the shaft, it is necessary to limit the reduction in the cross-sectional area of the space between the shaft portion of the catheter and the inner tube so as to prevent shutting off the flow of fluid to the balloon portion.

As will be clear to those skilled in the art, the balloon catheter of the invention is capable of substantial variation in its physical dimensions as well as in its use. Also, the materials used in construction of the catheter may be varied from those used in the working example so long as the rules of biological compatibility are observed.

It will thus be seen that the objects set forth above, among those made apparent from the preceding description, are efficiently attained and, since certain changes may be made in the above article without departing from the spirit and scope of the invention, it is intended that all matters contained in the above description and shown in the accompanying drawings shall be interpreted as illustrative and not in a limiting sense.

## Claims

1. A dilatation catheter having a balloon portion (I) which expands when the catheter is pressurized, the catheter comprising:

a shaft (4) of elastomeric material reinforced with filaments, a portion (I) of the shaft being capable when internally pressurized of expansion in diameter to provide a balloon, the filaments of the balloon portion lying at an angle which is less than a predetermined critical angle relative to the axis, whereby, when the balloon portion is expanded under pressure, expansion of the balloon stops when the filaments lie at the critical angle; and

an axially extendible portion (II) in which the filaments lie at an angle which is greater than the critical angle, the axially extendible portion increasing in length when the catheter is pressurized.

2. The dilatation catheter of claim 1, in which the shaft further comprises:

an immovable portion in which the filaments lie at the critical angle, the length of the immovable portion remaining constant when the catheter is pressurized.

3. The dilatation catheter of claim 1 or claim 2, in which the length of the balloon portion decreases when the catheter is pressurized and the resulting decrease in length is offset, at least in part, by an increase in length of the axially extendible portion.

4. The dilatation catheter of claim 3, in which the decrease in length of the balloon portion of the catheter is offset by the increase in length of the axially extendible portion of the catheter, whereby the position of the balloon portion does not change when the balloon is expanded.

5. The dilatation catheter according to any preceding claim, in which the balloon portion is formed at the distal end of the shaft and the axially extendible portion lies adjacent thereto.

6. The dilatation catheter of any preceding claim, further including:

inner tube means coaxially disposed inside the shaft, the inner tube means sealed to the distal end of the shaft to provide an annular space for receiving fluid under pressure between the shaft and the inner tube means.

7. The dilatation catheter of claim 6, further including:

connector means to which the inner tube means and the shaft are sealed, the connector maintaining the inner tube means and the shaft in coaxial relationship; and

passageway means in the connector means for passing fluid under pressure into the space between the inner tube means and the shaft.

8. The dilatation catheter of claim 6 or claim 7, in which the distal end of the balloon portion of the shaft is fixed to the distal end of the inner tube means.

9. The dilatation catheter of any of claims 6—8, in which the inner tube means comprises an elongated axial passageway for use in threading the catheter onto a guide wire.

10. The dilatation catheter of any preceding claim, in which the diameter of the unpressurized shaft is substantially uniform along the full length.

11. The dilatation catheter of any of claims 6—10, in which, when pressurized, the reduced diameter of the axially extendible portion of the shaft remains greater than the diameter of the inner tube means so as to permit the passage of fluid under pressure into and out of the balloon portion.

12. The dilatation catheter of any of claims 6—11, in which the inner tube means comprises polytetrafluoroethylene, and in which the filamentary material is embedded in polyurethane.

13. The dilatation catheter of any preceding claim, in which the shaft is formed of biologically compatible elastic coating means on the filaments of the balloon portion.

14. The dilatation catheter of claim 13, in which the filaments comprise nylon.

15. The dilatation catheter of any preceding claim, in which the filaments of the shaft comprise a tubular braid.

16. A balloon catheter assembly for introduction into a blood vessel, comprising:

an inner tube (26);

an outer tube (4) in coaxial relation to the inner tube;

the inner diameter of the outer tube being larger than the outer diameter of the inner tube so that the two tubes are axially displaceable with respect to each other and form an annular space between the tubes, and the distal end (24) of the inner tube extending beyond the distal end (25) of the outer tube;

the outer tube formed of an elastomeric material reinforced by filamentary material and having at least an inflatable balloon portion (I) and an axially extendible portion (II);

the filamentary material in the balloon portion lying at an angle which is less than a critical angle relative to the axis so that when fluid is introduced under pressure into the annular space between the tubes, the balloon expands in diameter until the filamentary material is extended at the critical angle; and

the filamentary material in the axially extendible portion lying at an angle greater than the critical angle so that the elongation portion expands in length in response to the pressure of the fluid;

whereby a balloon forms without any overall length reduction of the assembly.

17. A balloon catheter assembly for introduction into a blood vessel, comprising:

a catheter guide wire (55) having a distal end and a proximal end;

a catheter shaft (4) having a distal end (25) and a proximal end, the shaft of elastomeric material reinforced with filaments, a portion (I) of the shaft being capable, when internally pressurized, of expansion in diameter to provide a balloon, the filaments of the balloon portion lying at an angle which is less than a predetermined critical angle relative to the axis, whereby, when the balloon portion is expanded under pressure, expansion of the balloon stops when the filaments lie at the critical angle; and

the cathether shaft further including an axially extendible portion (II) in which the filaments lie at an angle which is greater than the critical angle, the axially extendible portion increasing in length when the catheter is pressurized.

18. The balloon catheter of claim 21, wherein the shaft is fixed to the guide wire in the region of the distal end of the wire.

**Patentansprüche**

1. Dehnungskatheter mit einem bei Unterdrucksetzung des Katheters expandierenden Ballonabschnitt (I), wobei der Katheter einen Schaft (4) aus mit Fasern verstärktem elastomeren Werkstoff aufweist, wobei ein Abschnitt (I) des Schafts bei innerer Unterdrucksetzung in seinem Durchmesser expandieren kann, so daß er einen Ballon darstellt, wobei die Fasern des Ballonabschnitts in einem Winkel zur Achse orientiert sind, der kleiner als ein vorgegebener kritischer Winkel ist, wodurch, wenn der Ballonabschnitt unter Druck expandiert wird, die Expansion des Ballonabschnitts anhält, wenn die Fasern in dem kritischen Winkel orientiert sind, wobei der Katheter des weiteren einen axial dehnbaren Abschnitt (II) aufweist, in dem die Fasern in einem Winkel orientiert sind, der größer als der kritische Winkel ist, und wobei der axial dehnbare Abschnitt in seiner Länge zunimmt, wenn der Katheter unter Druck gesetzt wird.

2. Dehnungskatheter nach Anspruch 1, bei dem der Schaft weiterhin einen nicht bewegbaren Abschnitt aufweist, in dem die Fasern im kritischen Winkel orientiert sind, wobei die Länge des unbewegbaren Abschnitts bei Unterdrucksetzung des Katheters konstant bleibt.

3. Dehnungskatheter nach Anspruch 1 oder 2, bei dem die Länge des Ballonabschnitts abnimmt, wenn der Katheter unter Druck gesetzt wird, wobei die sich ergebende Längenabnahme zumindest teilweise durch einen Anstieg der Länge des axial dehnbaren Abschnitts ausgeglichen wird.

4. Dehnungskatheter nach Anspruch 3, bei dem die Längenabnahme des Ballonabschnitts des Katheters durch die Längenzunahme des axial dehnbaren Abschnitts des Katheters ausgeglichen wird, so daß sich die Stellung des Ballonabschnitts während der Expansion des Ballons nicht ändert.

5. Dehnungskatheter nach einem der vorangehenden Ansprüche, bei dem der Ballonabschnitt am distalen Ende des Schafts gebildet ist und der axial dehnbare Abschnitt sich an den Ballonabschnitt anschließt.

6. Dehnungskatheter nach einem der vorangehenden Ansprüche, der weiterhin ein Innenschlauchelement aufweist, das koaxial innerhalb des Schafts angeordnet ist, wobei das Innenschlauchelement zum distalen Ende des Schafts abgedichtet ist, um einen Ringraum zur Aufnahme von Druckflüssigkeit zwischen dem Schaft und dem Innenschlauchelement zu schaffen.

7. Dehnungskatheter nach Anspruch 6, der weiterhin ein Verbindungsmittel aufweist, an dem das Innenschlauchelement und der Schaft abdichtend angebracht sind, wobei das Verbindungselement das Innenschlauchelement und den Schaft in koaxialer Anordnung hält, und wobei im Verbindungselement ein Durchlaß für Druckflüssigkeit in dem Raum zwischen dem Innenschlauchelement und dem Schaft vorgesehen ist.

8. Dehnungskatheter nach Anspruch 6 oder 7, bei dem das distale Ende des Ballonabschnitts des Schafts an dem distalen Ende des Innenschlauchelements befestigt ist.

9. Dehnungskatheter nach einem der Ansprüche 6—8, bei dem das Innenschlauchelement einen länglichen axialen Durchlaß zur Anwendung beim Aufwinden des Katheters auf einen Aufwinder aufweist.

10. Dehnungskatheter nach einem der vorangehenden Ansprüche, bei dem der Durchmesser des drucklosen Schafts über die gesamte Länge etwa gleich ist.

11. Dehnungskatheter nach einem der Ansprüche 6—10, bei dem nach Unterdrucksetzung der reduzierte Durchmesser des axial dehnbaren Abschnitts des Schafts größer als der Durchmesser des Innenschlauchelements bleibt, so daß der Durchlauf von Druckflüssigkeit in und aus dem Ballonabshnitt möglich ist.

12. Dehnungskatheter nach einem der Ansprüche 6—11, bei dem das Innenschlauchelement Polytetrafluoroethylene aufweist und bei dem der faserartige Werkstoff in Polyurethane eingebettet ist.

13. Dehnungskatheter nach einem der vorangehenden Ansprüche, bei dem der Schaft auf den Fasern des Ballonabschnitts aus einem biologisch verträglichem elastischem Schichtelement gebildet ist.

14. Dehnungskatheter nach Anspruch 13, bei dem die Fasern Nylon aufweisen.

15. Dehnungskatheter nach einem der vorangehenden Ansprüche, bei dem die Fasern des Schafts eine zylindrische Wicklung bilden.

16. Ballonkatheteranordnung zur Einführung in ein Blutgefäß mit einem Innenschlauch (26), einem Außenschlauch (4), der in koaxialer Anordnung zum Innenschlauch ist, wobei der Innendurchmesser des Außenschlauchs größer als der Außendurchmesser des Innenschlauchs ist, so daß die beiden Schläuche relativ zueinander axial versetzbar sind und einen Ringraum zwischen den Schläuchen ausbilden, und wobei das distale Ende (24) des Innenschlauchs sich über das distale Ende (25) des Außenschlauchs erstreckt, wobei der Außenschlauch aus einem mit faserigem Werkstoff verstärkten elastomeren Werkstoff gebildet ist und zumindest einen aufblasbaren Ballonabschnitt (I) und einen axial dehnbaren Abschnitt (II) aufweist, wobei das faserige Material im Ballonabschnitt in einem Winkel in bezug auf die Achse orientiert ist, der kleiner als ein kritischer Winkel ist, so daß, wenn Fluid unter Druck in den Ringraum zwischen den Schläuchen eingeführt wird, der Ballon im Durchmesser expandiert, bis das faserige Material im kritischen Winkel ausgerichtet ist, und wobei das faserige Material in dem axial dehnbaren Abschnitt in einem Winkel orientiert ist, der größer als der kritische Winkel ist, so daß sich der axial dehnbare Abschnitt in seiner Länge entsprechend dem Druck des Fluids ausdehnt, wodurch sich ein Ballon ohne Gesamtlängenreduzierung der Anordnung bildet.

17. Ballonkatheteranordnung zur Einführung in ein Blutgefäß mit einem Katheteraufwinder (55) mit einem distalen Ende und einem proximalen Ende, einem Katheterschaft (4) mit einem distalen Ende (25) und einem proximalen Ende, wobei der aus elastomeren Werkstoff bestehende Schaft mit Fasern verstärkt ist, einem Abschnitt (I) des Schafts, der sich bei innerer Unterdrucksetzung im Durchmesser ausdehnen kann, um einen Ballon auszubilden, wobei die Fasern des Ballonabschnitts in bezug auf die Achse in einem Winkel angeordnet sind, der kleiner als ein vorgegebener kritischer Winkel ist, wodurch, wenn der Ballonabschnitt unter Druck expandiert, die Expansion des Ballonabschnitts anhält, wenn die Fasern im kritischen Winkel angeordnet sind, und wobei der Katheterschaft weiterhin einen axial dehnbaren Abschnitt (II) aufweist, in dem die Fasern in einem Winkel angeordnet sind, der größer als der kritische Winkel ist, so daß sich der axial dehnbare Abschnitt in seiner Länge vergrößert, wenn der Katheter unter Druck gesetzt wird.

18. Ballonkatheter nach Anspruch 17, bei dem der Schaft im Bereich des distalen Endes des Aufwinders am Aufwinder befestigt ist.

**Revendications**

1. Cathéter à dilatation comportant une partie (I) gonflable qui se dilate quand le cathéter est pressurisé, le cathéter comprenant:

—une tige (4) en matériau élastomère renforcé par des filaments, une partie (I) de la tige étant susceptible lorsqu'elle est pressurisée intérieurement de se dilater en diamètre pour former un ballon, les filaments de la partie gonflable étant orientés sous un angle qui est inférieur à un angle critique prédéterminé par rapport à l'axe, de façon qu'au moment où la partie gonflable est dilatée sous pression, la dilatation du ballon s'arrête quand les filaments arrivent sous l'angle critique; et

—une partie (II) extensible dans la direction axiale dans laquelle les filaments sont orientés sous un angle supérieur à l'angle critique, la partie extensible dans la direction axiale augmentant en longueur lorsque le cathéter est pressurisé.

2. Cathéter à dilatation selon la revendication 1, dans lequel la tige comporte en outre une partie inamovible dans laquelle les filaments sont orientés sous l'angle critique, la longueur de la partie inamovible restant constante quand le cathéter est pressurisé.

3. Cathéter à dilatation selon la revendication 1 ou 2, dans lequel la longueur de la partie gonflable diminue lorsque le cathéter est pressurisé et dans lequel la diminution résultante de longueur est compensée, au moins en partie, par une augmentation de la longueur de la partie extensible dans la direction axiale.

4. Cathéter à dilatation selon la revendication 3, dans lequel la réduction de longueur de la partie gonflable du cathéter est compensée par une augmentation en longueur de la partie extensible du cathéter dans la direction axiale, de façon que la position de la partie gonflable ne varie pas quand le ballon est dilaté.

5. Cathéter à dilatation selon l'une quelconque des revendications précédentes, dans lequel la partie gonflable est formée à l'extrémité distale de la tige et la partie extensible dans la direction axiale est adjacente à cette dernière.

6. Cathéter à dilatation selon l'une quelconque des revendications précédentes, comprenant en outre un moyen de tube intérieur disposé coaxialement à l'intérieur de la tige, le moyen de tube intérieur étant fermé par rapport à l'extrémité distale de la tige pour créer un espace annulaire destiné à recevoir un fluide sous pression entre la tige et le moyen de tube intérieur.

7. Cathéter à dilatation selon la revendication 6, comprenant en outre:
—un moyen de connecteur auquel le moyen de tube intérieur et la tige sont scellés, le connecteur maintenant le moyen de tube intérieur et l'arbre en relation coaxiale; et
—un moyen de passage dans le moyen de connecteur pour laisser passer un fluide sous pression dans l'espace entre le moyen de tube intérieur et la tige.

8. Cathéter à dilatation selon la revendication 6 ou 7, dans lequel l'extrémité distale de la partie gonflable de la tige est fixée à l'extrémité distale du moyen de tube intérieur.

9. Cathéter à dilatation selon l'une quelconque des revendications 6 à 8, dans lequel le moyen de tube intérieur comporte un passage axial allongé à utiliser dans la mise en place du cathéter sur un fil de guidage.

10. Cathéter à dilatation selon l'une quelconque des revendications précédentes, dans lequel le diamètre de la tige non pressurisée est sensiblement uniforme sur toute sa longueur.

11. Cathéter à dilatation selon l'une quelconque des revendications 6 à 10, dans lequel, lorsqu'il est pressurisé, le diamètre réduit de la partie de la tige extensible dans la direction axiale reste supérieur au diamètre du moyen de tube intérieur afin de permettre le passage d'un fluide sous pression entrant et sortant de la partie gonflable.

12. Cathéter à dilatation selon l'une quelconque des revendications 6 à 11, dans lequel le moyen de tube intérieur est en polytétrafluoréthylène, et dans lequel le matériau des filaments est enrobé dans du polyuréthane.

13. Cathéter à dilatation selon l'une quelconque des revendications précédentes, dans lequel la tige est constituée d'un moyen de revêtement élastique à compatibilité biologique sur les filaments de la partie gonflable.

14. Cathéter à dilatation selon la revendication 13, dans lequel les filaments sont en nylon.

15. Cathéter à dilatation selon l'une quelconque des revendications précédentes, dans lequel les filaments de la tige constituent une tresse tubulaire.

16. Assemblage de cathéter gonflable destiné à être introduit dans un vaisseau sanguin, comprenant:
—un tube intérieur (26);
—un tube extérieur (4) en relation coaxiale avec le tube intérieur;
le diamètre intérieur du tube extérieur étant supérieur au diamètre extérieur du tube intérieur afin que les deux tubes puissent être déplacés l'un par rapport à l'autre dans la direction axiale et forment un espace annulaire entre les tubes, et dans lequel l'extrémité distale (24) du tube intérieur s'étend au-delà de l'extrémité distale (25) du tube extérieur;
le tube extérieur constitué d'un matériau élastomère renforcé par des matériaux en forme de filaments et comprenant au moins une partie (I) gonflable et une partie (II) extensible dans la direction axiale;
le materiau des filaments de la partie gonflable étant orienté sous un angle inférieur à un angle critique par rapport à l'axe afin qu'au moment où un fluide est introduit sous pression dans l'espace annulaire entre les tubes, le ballon augmente de diamètre jusqu'à ce que le matériau des filaments soit orienté sous l'angle critique; et
le matériau des filaments de la partie extensible dans la direction axiale étant orienté sous un angle supérieur à l'angle critique afin que la partie extensible en longueur s'allonge en réponse à la pression du fluide;
de sorte qu'un ballon se forme sans réduire la longueur totale de l'ensemble.

17. Assemblage de cathéter gonflable à introduire dans un vaisseau sanguin, comprenant:
—un fil (55) de guidage du cathéter ayant une extrémité distale et une extrémité proximale;
—une tige de cathéter (4) ayant une extrémité distale (25) et une extrémité proximale, la tige en matériau élastomère renforcé par des filaments, une partie (I) de la tige étant capable, lorsqu'elle est pressurisée intérieurement, de se dilater en diamètre pour former un ballon, les filaments de la partie gonflable étant orientés sous un angle qui est inférieur à un angle critique prédéterminé par rapport à l'axe, de façon qu'au moment où la partie gonflable est dilatée sous pression, la dilatation du ballon s'arrête quand les filaments arrivent sous l'angle critique; et
la tige du cathéter comprenant en outre une partie (II) extensible dans la direction axiale dans laquelle les filaments sont orientés sous un angle qui est supérieur à l'angle critique, la partie extensible dans la direction axiale augmentant en longueur quand le cathéter est pressurisé.

18. Cathéter gonflable selon la revendication 21, dans lequel la tige est fixée au fil de guidage dans la région de l'extrémité distale du fil.

FIG.1

FIG. 2a

FIG. 2b

EP 0 240 504 B1

EP 0 240 504 B1

I   II   III

$\beta$

4

## FIG.3a

I   II   III

$\alpha$   $\alpha$   $\alpha$

4

## FIG.3b

$\delta_{CIRC}$

$h_b$

$\pi D_B$

$\pi d$

$\alpha$   $\beta$

$\delta_{AXIAL}$

$P_{Bi}$

$\Delta P_B$

$P_{Bd}$

## FIG.5a

$\delta_{CIRC}$

$h_s$

$\pi ds$

$\pi D_S$

$\alpha$   $\sigma$

$\delta_{AXIAL}$

$P_{Sd}$

$\Delta P_S$

$P_{Si}$

## FIG.5b

FIG.4a

FIG.4b

FIG.4c

FIG.6